# EUROPEAN PATENT APPLICATION

(11) **EP 2 447 195 A1**
(43) Date of publication of application: **02.05.2012**
(21) Application number: 10791804.7
(22) Date of filing: 14.06.2010
(51) Int. Cl.: B65G 47/29, B65C 3/12, B65C 3/14, B65C 9/46, B65G 47/10, B65G 47/53, G01N 35/04

(54) **BLOOD COLLECTION TUBE STOCKER AND DEVICE FOR PREPARING BLOOD COLLECTION TUBES**

(30) Priority: 24.06.2009 JP 2009167245; 09.09.2009 JP 2009207765; 02.11.2009 JP 2009251674
(71) Applicant: Afc Limited, Osaka 553-0003 (JP)
(72) Inventor: TAKAHASHI, Nobuo, Chiba-shi Chiba 261-0011 (JP)
(74) Representative: Mischung, Ralf
(86) International application number: PCT/JP2010/003943
(87) International publication number: WO 2010/150479

(57) **Abstract**

Provided is a blood collection tube storage box which performs an operation of preparing a blood collection tube in advance in a blood collection step as a pre-step of a blood collection test. The blood collection tube storage box includes a transferring belt (2), which is brought into abutment with an outer peripheral side surface of a blood collection tube main body (4A) to transfer the blood collection tube main body (4A), a side surface support plate (11), which is brought into point contact with an outer peripheral side surface of the blood collection tube main body (4A) on an opposite side of the transferring belt (2) to support the blood collection tube main body (4A) so that the side surface support plate (11) is opposed to the transferring belt (2), and an inclined support plate (3), which is brought into point contact with a lower end surface edge portion of a blood collection tube cap (10) on the transferring belt (2) side to support the blood collection tube cap (10) in a suspended manner. The above-mentioned mechanism portions are configured as an easily-detachable module. The form of the module allows the storage box to be simplified and downsized, and work in a case of a failure and work for maintenance to be greatly facilitated.

## Description

### Technical Field

The present invention relates to a blood collection tube storage box and a blood collection tube preparing apparatus which perform an operation of preparing a blood collection tube in advance in a blood collection step as a pre-step of a blood collection test. The blood collection tube storage box includes a transferring belt, which is brought into abutment with an outer peripheral side surface of a blood collection tube main body to transfer the blood collection tube main body, a side surface support plate, which is brought into point contact with an outer peripheral side surface of a blood collection tube on an opposite side of the transferring belt to support the blood collection tube (in a horizontal direction), and an inclined support plate, which is brought into point contact with a lower end surface edge of a blood collection tube cap on the opposite side of the transferring belt to support the blood collection tube (in a vertical direction) in a suspendedmanner. The blood collection tube preparing apparatus of an all-in-one type uses the blood collection tube storage box.

### Background Art

In a blood test department of a hospital, a clinic, or another medical institution, a blood collection operator (such as nurse) collects and stores blood of a patient into a blood collection tube, and then transfers the blood collection tube to the test department. In a blood test, a wide variety of blood collection tubes are prepared correspondingly to blood test items. Normally, a plurality of kinds of tests are performed on one patient at the same time. Therefore, a plurality of kinds of blood collection tubes are automatically prepared for one patient by the blood collection tube preparing apparatus. Further, it is common to treat a large number of patients in a blood collection room, and a label, on which barcodes containing patient information, blood collection test information, and the like are printed, is stuck onto each of the blood collection tubes prepared by the blood collection tube preparing apparatus.
Further, when a patient comes to a reception desk to undergo blood collection, a reception ticket for blood collection, on which barcodes containing a reception number and the like are printed, is issued for the patient. In blood collection operation, the contents of the label and the reception ticket are read by an optical device such as a barcode reader for the purpose of preventing confusion of patients or blood collection tubes.

As a technology for achieving automation of blood collection operation as described above and for supporting the blood collection operation, there is known a technology disclosed in Patent Literature 1. In the technology disclosed in Patent Literature 1, a test tube holding member is provided between reciprocating belts of one endless transferring belt that is driven to rotate in a horizontal direction. The test tube holding member is a guide frame having a smooth surface such as a rail-like or plate-like member and supports one side of a cap lower end surface of a test tube. An opposing lower end surface on the other side of the cap is supported on an upper end surface of a forward-side transferring belt. Thus, the test tube moves sliding over the test tube holding member and the forward-side transferring belt with the cap lower end surface supported by the test tube holding member and the upper end surface of the forward-side transferring belt. The test tube is transferred by the joint between the upper end surface of the forward-side transferring belt and the lower end surface of the test tube cap. The transferring belt has a slight gap with respect to the outer peripheral side surface of the test tube and is not in contact therewith.
On the terminal end side of the test tube holding member, the test tube is disengaged from the test tube holding member to be cantilever-supported on the upper end surface of the forward-side transferring belt. Then, the cap (head) side is inclined toward the test tube holding member disengaged from the test tube so that the test tube falls down. Thereafter, the test tube is discharged.

Thus, according to the conventionally known invention, a hanger-type storage box, which supports the cap lower end surface of the test tube, is configured in the reciprocating belts of one conveying belt. Therefore, one conveying device for test tubes in the storage box can be configured for one kind of test tubes, which can minimize the number of conveying devices. Further, the storage box for test tubes is configured in the reciprocating belts of one conveying belt, and hence, the width of one storage box is merely several centimeters. Thus, there is an advantage that, even in the case where a plurality of kinds of storage boxes are provided so as to handle a plurality of kinds of test tubes, the entire width dimension can be remarkably reduced compared with that of the prior art.
Patent Literature 1: JP 4356096 B

### Summary of Invention

### Technical Problems

However, in the technology disclosed in Patent Literature 1, the drive force of the conveying belt is transmitted to the cap lower end surface of the blood collection tube disposed on the upper end surface through the upper end surface of the forward-side conveying belt. On the other hand, the other side of the lower end surface of the blood collection tube cap moves sliding over a support rail. Therefore, when a friction resistance is large between the support rail and the cap lower end surface, the friction resistance becomes larger than the drive force for conveying the blood collection tube, which is transmitted from the upper end surface of the forward-side conveying belt to the lower end surface of the blood collection tube cap. Consequently, slippery occurs between the lower end surface of the blood collection tube cap and the upper end surface of the conveying belt, which raises a problem that the blood collection tube cannot be conveyed.
This problem is serious in a rubber cap blood collection tube having a large friction coefficient, which raises a problem that the rubber cap blood collection tube cannot be conveyed in any way.

Further, in the conventional technology disclosed in Patent Literature 1, the blood collection tube cap lower end surface is supported by the support rail on one side and supported by the upper end surface of the conveying belt on the other side, and the conveying belt transfers the blood collection tube directly by the abutment with the cap lower end surface. Therefore, there is a problem of durability caused by the abrasion due to the contact between the cap lower end surface and the end surface of the conveying belt, and further, there is a risk that the conveying belt may be twisted to attract the cap of the blood collection tube to the belt, with the result that the blood collection tube falls down.

Further, there is also a seal-like cap having an outer diameter dimension slightly larger than that of the blood collection tube. However, in this case, there is a problem that the cap lower end surface has no room of a required width dimension for being disposed on the end surface of the conveying belt, and thus, the use of the seal-like cap is not practical.

In view of the above-mentioned conventional problems, the present invention has been achieved by improving the conventional example and solving the problems thereof, and has an object to provide a blood collection tube storage box including a transferring belt, which is brought into abutment with an outer peripheral side surface of a main body of a blood collection tube (including a test tube) to transfer the blood collection tube main body, a side surface support plate, which is brought into point contact with an outer peripheral side surface of the blood collection tube main body on an opposite side of the transferring belt to support the blood collection tube main body, and an inclined support plate, which is brought into point contact with a lower end surface edge of a blood collection tube cap on the opposite side of the transferring belt to support the blood collection tube in a suspended manner.
Further, the present invention has an object to provide a blood collection tube preparing apparatus with a downsized and simple structure excellent in mobility, capable of selecting various kinds of blood collection tubes from the storage box depending on a blood correction instruction from a doctor, sticking labels printed with blood collection information such as patient IDs and contents of the blood collection instruction, and receiving the blood collection tube, to which the label has been stuck, in a tray for each patient.

### Solution to Problems

Therefore, in order to solve the above-mentioned problems, as means of claim 1 adopted in the present invention, there is provided a blood collection tube storage box, including: an endless transferring belt that is looped around pulleys provided on both end sides of the endless transferring belt and is driven to rotate in a horizontal direction, the endless transferring belt being brought into abutment with an outer peripheral side surface of a blood correction tube to transfer the blood collection tube; a side surface support plate having an end surface partially brought into point contact with an outer peripheral side surface of the blood collection tube on an opposite side of the endless transferring belt to support the blood collection tube; an inclined support plate brought into point contact with a lower end surface edge of a cap of the blood collection tube on the opposite side of the endless transferring belt to support the blood correction tube in a suspended manner, the side surface support plate and the inclined support plate being positioned between reciprocating belts of the endless transferring belt and above an upper end of the endless transferring belt; a discharging portion through which the blood collection tube falls down by an own weight of the blood collection tube to be discharged, the discharging portion being provided at a position at which the blood collection tube passes a terminal end side of the inclined support plate that supports the blood collection tube in the suspended manner; a first stopper capable of moving close to and away from a front surface of a cap of a leading blood collection tube; a second stopper capable of moving close to and away from a gap from above, the gap being formed between the cap of the leading blood collection tube and a cap of a second blood collection tube, the first stopper and the second stopper being provided on the terminal end side of the inclined support plate; two arms which are pivotally supported by the same shaft, and to which the first stopper and the second stopper are fixed on distal end sides of the two arms, respectively; and solenoids coupled on trailing end sides of the two arms, respectively.

As means of claim 2 adopted in the present invention, there is provided a blood collection tube storage box, including: an endless transferring belt that is looped around pulleys provided on both end sides of the endless transferring belt and is driven to rotate in a horizontal direction, the endless transferring belt being brought into abutment with an outer peripheral side surface of a blood correction tube to transfer the blood collection tube; a side surface support plate having an end surface partially brought into point contact with an outer peripheral side surface of the blood collection tube on an opposite side of the endless transferring belt to support the blood collection tube; an inclined support plate brought into point contact with a lower end surface edge of a cap of the blood collection tube on the opposite side of the endless transferring belt to support the blood correction tube in a suspended manner, the side surface support plate and the inclined support plate being positioned between reciprocating belts of the endless transferring belt and above an upper end of the endless transferring belt; a discharging portion through which the blood collection tube falls down by an own weight of the blood collection tube to be discharged, the discharging portion being provided at a position at which the blood collection tube passes a terminal end side of the inclined support plate that supports the blood collection tube in the suspended manner; a first stopper capable of moving close to and away from a front surface of a cap of a leading blood collection tube; a second stopper capable of moving close to and away from a gap from above, the gap being formed between the cap of the leading blood collection tube and a cap of a second blood collection tube, the first stopper and the second stopper being provided on the terminal end side of the inclined support plate; two arms which are pivotally supported by the same shaft, and to which the first stopper and the second stopper are fixed on distal end sides of the two arms, respectively; and solenoids coupled on trailing end sides of the two arms, respectively, the endless transferring belt, the inclined support plate, the discharging portion for the blood collection tube, the first stopper, the second stopper, the two arms, and the solenoids being accommodated in one casing, to thereby form a module.

As means of claim 3 adopted in the present invention, there is provided a blood collection tube preparing apparatus, including: an endless transferring belt that is looped around pulleys provided on both end sides of the endless transferring belt and is driven to rotate in a horizontal direction, the endless transferring belt being brought into abutment with an outer peripheral side surface of a blood correction tube to transfer the blood collection tube; a side surface support plate having an end surface partially brought into point contact with an outer peripheral side surface of the blood collection tube on an opposite side of the endless transferring belt to support the blood collection tube; an inclined support plate brought into point contact with a lower end surface edge of a cap of the blood collection tube on the opposite side of the endless transferring belt to support the blood correction tube in a suspended manner, the side surface support plate and the inclined support plate being positioned between reciprocating belts of the endless transferring belt and above an upper end of the endless transferring belt; a discharging portion through which the blood collection tube falls down by an own weight of the blood collection tube to be discharged, the discharging portion being provided at a position at which the blood collection tube passes a terminal end side of the inclined support plate that supports the blood collection tube in the suspended manner; a first stopper capable of moving close to and away from a front surface of a cap of a leading blood collection tube; a second stopper capable of moving close to and away from a gap from above, the gap being formed between the cap of the leading blood collection tube and a cap of a second blood collection tube, the first stopper and the second stopper being provided on the terminal end side of the inclined support plate; two arms which are pivotally supported by the same shaft, and to which the first stopper and the second stopper are fixed on distal end sides of the two arms, respectively; solenoids coupled on trailing end sides of the two arms, respectively; a conveying belt for the blood collection tube, the conveying belt being provided below the discharging portion; label printing/sticking means provided on a terminal end side of the conveying belt; and a tray disposing portion for receiving the blood collection tube, the tray disposing portion being provided in a vicinity of a delivery portion of the label printing/sticking means.

As means of claim 4 adopted in the present invention, there is provided a blood collection tube preparing apparatus, including: a plurality of blood collection tube storage boxes, each of the plurality of blood collection tube storage boxes including: an endless transferring belt that is looped around pulleys provided on both end sides of the endless transferring belt and is driven to rotate in a horizontal direction, the endless transferring belt being brought into abutment with an outer peripheral side surface of a blood correction tube to transfer the blood collection tube; a side surface support plate having an end surface partially brought into point contact with an outer peripheral side surface of the blood collection tube on an opposite side of the endless transferring belt to support the blood collection tube; an inclined support plate brought into point contact with a lower end surface edge of a cap of the blood collection tube on the opposite side of the endless transferring belt to support the blood correction tube in a suspended manner, the side surface support plate and the inclined support plate being positioned between reciprocating belts of the endless transferring belt and above an upper end of the endless transferring belt; a discharging portion through which the blood collection tube falls down by an own weight of the blood collection tube to be discharged, the discharging portion being provided at a position at which the blood collection tube passes a terminal end side of the inclined support plate that supports the blood collection tube in the suspended manner; a first stopper capable of moving close to and away from a front surface of a cap of a leading blood collection tube; a second stopper capable of moving close to and away from a gap from above, the gap being formed between the cap of the leading blood collection tube and a cap of a second blood collection tube, the first stopper and the second stopper being provided on the terminal end side of the inclined support plate; two arms which are pivotally supported by the same shaft, and to which the first stopper and the second stopper are fixed on distal end sides of the two arms, respectively; and solenoids coupled on trailing end sides of the two arms, respectively, the endless transferring belt, the blood collection tube supporting member, the discharging portion for the blood collection tube, the first stopper, the second stopper, the two arms, and the solenoids being accommodated in a module serving as one casing, the plurality of blood collection tube storage boxes being detachably and respectively received, in a form of the modules, in a plurality of lanes aligned in parallel to each other; a conveying belt provided below a discharging section ranging over a region of the discharging portion of the each of the plurality of blood collection tube storage boxes; label printing/sticking means provided on a terminal end side of the conveying belt; and a tray disposing portion for receiving the blood collection tube, the tray disposing portion being provided in a vicinity of a delivery portion of the label printing/sticking means.

As means of claim 5 adopted in the present invention, there is provided a blood collection tube preparing apparatus according to claim 3 or 4, further including an opening portion which is formed in a vicinity of the discharging portion of one of the plurality of blood collection tube storage boxes situated on a most upstream side of the conveying belt, and which allows the blood collection tube to be manually inserted onto the conveying belt through the opening portion.

As means of claim 6 adopted in the present invention, there is provided a blood collection tube preparing apparatus according to any one of claims 3 to 5, further including a setting state display portion provided on an outer surface of a blood collection tube preparing apparatus main body, for displaying a setting state of the blood collection tube preparing apparatus and displaying guidance for changing settings in association with each operation switch.

### Advantageous Effects of Invention

In the invention according to claim 1, the inclined support plate for holding the blood collection tube in the suspended manner is provided between the reciprocating belts of one endless transferring belt that is driven to rotate in the horizontal direction. The inclined support plate is a guide frame having a smooth surface such as a rail-like or plate-like member, and supports an edge on one side of a cap lower end surface of the blood collection tube by point contact. Below the inclined support plate, the side surface support plate that is brought into abutment with the outer peripheral side surface of the blood collection tube to support the blood collection tube is provided. The inclined support plate and the side surface support plate can also be produced by bending one continuous flat plate.
Further, the endless transferring belt is in abutment with the outer peripheral side surface of the blood collection tube on the opposite side of the side on which the inclined support plate and the side surface support plate are provided. The kind of the transferring belt is not limited. However, it is preferred that round belts that are more excellent in the transmission of a drive force to the blood collection tube, compared with that of a flat belt, be provided in a plurality of stages in a vertical direction.
In the vertical direction, the blood collection tube is supported with an edge portion on the lower end surface on one side of a blood collection tube cap engaged with the inclined support plate by point contact. Further, in a horizontal direction, on the side where the inclined support plate is arranged, a leading end edge portion of the bent side surface support plate is in point contact with the outer peripheral side surface of the blood collection tube. On the other hand, on the opposite side of the side where the inclined support plate is arranged, the transferring belt is in abutment with the outer peripheral side surface of a blood collection tube main body with some urging force.

Thus, the blood collection tube is supported in the horizontal direction by the transferring belt and the side surface support plate while being pressed against the edge portion of the side surface support plate with the urging force of the transferring belt. Further, the transferring belt transfers the blood collection tube through the contact with the outer peripheral side surface of the blood collection tube. Therefore, the blood collection tube slides over the inclined support plate with the lower end surface edge portion of the blood collection tube cap in point contact with the inclined support plate, and moves sliding with the edge portion of the side surface support plate in point contact with the outer peripheral side surface of the blood collection tube. As described above, the blood collection tube is supported by the respective point contacts of the inclined support plate and the side surface support plate, and hence, the friction force between the supporting members and the blood collection tube is very small. Therefore, the drive force to be transmitted from the transferring belt to the blood collection tube is larger than the friction force for supporting the blood collection tube in a point contact method, which enables smooth conveyance and also realizes the conveyance of a rubber cap tube.
On the terminal end side of the inclined support plate and the side surface support plate, the blood collection tube is disengaged therefrom and falls down. Then, the blood collection tube is discharged.

Further, according to the invention of claim 1, there is adopted a mechanism for discharging the leading blood collection tube through isolating the leading blood collection tube from the other blood collection tubes, and the first stopper and the second stopper are provided. The first stopper is capable of moving close to and away from the front surface side of the cap of the leading blood collection tube so as to abut against the same. The second stopper is capable of moving close to and away from the gap formed between the caps of the leading and second blood collection tubes.

The first stopper abuts against the front surface side of the cap of the leading blood collection tube, and the second stopper enters the gap formed between the caps of the leading and second blood collection tubes. Under this state, the first stopper is operated to move away from the leading blood collection tube, and to disengage from the front surface side of the cap of the leading blood collection tube. Then, the leading blood collection tube is further moved ahead through being transferred while the forward-side transferring belt is joined thereto. After a while, the terminal end side of the inclined support plate and the side surface support plate that support the blood collection tube is disengaged. Therefore, the leading blood collection tube falls down by its own weight to be discharged. The second and subsequent blood collection tubes remain stopped owing to the second stopper. After the leading blood collection tube is discharged, the first stopper is operated to return. When the second stopper is operated to move away from the gap, all of the remaining blood collection tubes are pushed to move ahead by a length of one blood collection tube, and the first stopper stops the movement of the remaining blood collection tubes. Then, the second stopper is operated to return to the original state.

In addition, according to the invention of claim 1, each of the first stopper and the second stopper is fixed to, for example, one end side of the arm, a middle portion of the arm is swingably and pivotally supported, and the solenoid (distal end of a rod of the solenoid) is coupled to the other end side of the arm. Therefore, through performing control to turn the solenoid ON and OFF, the rod of the solenoid is operated to protrude and retract with respect to a main body case, and each of the first stopper and the second stopper fixed on the one end side of the arm is operated to be capable of moving close to and away from the cap of the blood collection tube.
As described above, the first stopper and the second stopper are controlled and operated to move close to and away from the cap of the blood collection tube, and thus it is possible to discharge the leading blood collection tube through insolating the leading blood collection tube from the other blood collection tubes.

In the invention according to claim 2, the respective constituent members including the transferring belt, the inclined support plate, the side surface support plate, the discharging portion for the blood collection tube, the first and second stoppers, the two arms, and the solenoids constituting the blood collection tube storage box of claim 1 are housed in one casing, to thereby form a module. According to such a module, if modules corresponding to the kinds of the blood collection tubes are prepared, even in the case where there is a change in the tube kind of the blood collection tube, the module may merely be replaced, which enables a very simple and rapid operation. Further, in the case of failure, repair can be performed by a send-back method on the module basis, which makes it easy to handle remote areas.

The blood collection tube preparing apparatus according to the invention of claim 3 uses the blood collection tube storage box according to the invention of claim 1, and includes the conveying belt for conveying the blood collection tube discharged from the storage box through being isolated from the other blood collection tubes up to a position of the label printing/sticking means in a state in which the blood collection tube is laid so that the bottom side of the blood collection tube is directed forward, the label printing/sticking means provided on the terminal end side of the conveying belt, and the tray disposing portion for receiving the blood collection tube, the tray disposing portion being provided in the vicinity of the delivery portion of the label printing/sticking means. The label printing/sticking means obtains blood collection information of the blood collection patient from, for example, the upper system or a doctor's personal computer (PC) terminal, performs printing on a label, and sticks the printed label onto an outer peripheral surface of the blood collection tube. After being subjected to label sticking, the blood collection tube is received into the tray for one and the same patient.
With the above-mentioned blood collection tube preparing apparatus using the blood collection tube storage box, even when the blood collection tube preparing apparatus is structured to be capable of handling various kinds of blood collection tubes, the blood collection tube preparing apparatus has an extremely small size, and has an excellent characteristic in terms of lightweight and downsizing of the entire apparatus.

Therefore, the blood collection tube preparing apparatus according to the invention of claim 3 is compact and portable, and is easily moved. Accordingly, in the case of a disaster or the like, it is extremely easy to carry and place the blood collection tube preparing apparatus in an emergency facility or the like on the site. It is possible to capture information regarding blood collection performed on the patient that a doctor examines through connection to the computer terminal such as a laptop PC that the doctor uses, and to take the blood collection tube necessary for the blood collection out of the storage box selectively. Further, it is possible to receive the blood collection tube into the tray for each patient after sticking the printed label having the blood collection information of the patient onto the taken-out blood collection tube, and to automatically prepare for the blood collection. Further, there is an advantage that, for example, the blood collection tube preparing apparatus can be introduced also in a private hospital or the like. In addition, in in-patients wards, it is possible to bring the blood collection tube preparing apparatus on a movable wagon to the patient's bedside in each ward, to automatically prepare the blood collection tube of the apparatus, and to perform blood collection.

The blood collection tube preparing apparatus according to the invention of claim 4 includes the module-type blood collection tube storage box of the invention according to claim 2, the conveying belt for conveying the blood collection tube discharged from the storage box through being isolated from the other blood collection tubes up to a position of the label printing/sticking means in a state in which the blood collection tube is laid so that the bottom side of the blood collection tube is directed forward, the label printing/sticking means provided on the terminal end side of the conveying belt, and the tray disposing portion for receiving the blood collection tube, the tray disposing portion being provided in the vicinity of the delivery portion of the label printing/sticking means. Thus, the invention according to claim 4 has both the functional effect of a module and the functional effect of the invention according to claim 3.

According to the invention of claim 5, on the upstream side of the conveying belt for supplying, to the label printing/sticking means, the blood collection tube discharged from each of the blood collection tube storage boxes, the opening portion through which the blood collection tube can be manually put is provided. This is provided to treat the patient requiring a rarely-performed blood collection test or a special blood collection test. In each of the blood collection tube storage boxes, the blood collection tubes of a frequently-used tube kind are stored. In the case of the rarely-performed blood collection test or the special blood collection test, individual treatment is necessary. Thus, according to the invention of claim 5, the opening portion is provided to allow an operator to manually supply the blood collection tube used for the rarely-performed blood collection test or the special blood collection test, and the blood collection tube is supplied to the label printing/sticking means.

In the invention according to claim 6, the setting state display portion is provided on the outer surface of the apparatus main body. The setting state display portion displays the setting state of the apparatus and displays the guidance for changing settings in association with the function operation switch for each component of the apparatus main body. With this structure, it is possible to visually confirm the current setting state of the apparatus, and to display the contents to be changed and the like in the case of setting change.
Therefore, an operator can reliably confirm the setting state without any connection to an externally added device, and can advantageously perform the setting change.

### Brief Description of Drawings

[FIG. 1] A plan view illustrating an entire structure of a blood collection tube preparing apparatus according to an embodiment of the present invention.
[FIG. 2] A front view illustrating the entire structure of the blood collection tube preparing apparatus according to the embodiment of the present invention.
[FIG. 3] A back view illustrating the entire structure of the blood collection tube preparing apparatus according to the embodiment of the present invention.
[FIG. 4] A left-hand side view illustrating the entire structure of the blood collection tube preparing apparatus according to the embodiment of the present invention.
[FIG. 5] A plan view illustrating a basic structure of a blood collection tube storage box according to the embodiment of the present invention.
[FIG. 6] A side view illustrating an entire structure of the blood collection tube storage box according to the embodiment of the present invention.
[FIG. 7] A sectional view of the blood collection tube storage box according to the embodiment of the present invention taken along the line A-A of FIG. 5.
[FIG. 8] A partially enlarged view of FIG. 7, illustrating the blood collection tube storage box according to the embodiment of the present invention.
[FIG. 9] A plan view illustrating a first stopper and a second stopper according to the embodiment of the present invention.
[FIG. 10] A vertical sectional front view illustrating the blood collection tube preparing apparatus according to the embodiment of the present invention.
[FIG. 11] A vertical sectional view illustrating a fall-down discharge portion for a blood collection tube according to the embodiment of the present invention.
[FIG. 12] A side view illustrating positional relation among members constituting a label sticking device according to the embodiment of the present invention.

### Reference Signs List

- 1 ···: blood collection tube storage box
- 2: transferring belt
- 2A: forward-side transferring belt
- 2B: backward-side transferring belt
- 3: inclined support plate
- 4: blood collection tube
- 5: first stopper
- 5A: bar-like protrusion
- 6: second stopper
- 6A: bar-like protrusion
- 7: label sticking device
- 8A, 8B: pulley
- 10: cap
- 11: side surface support plate
- 38: setting state display portion
- A: blood collection tube preparing apparatus
- B: storage box placing portion
- C: placing portion for printing/sticking means
- D: placing portion D for conveying belt
- E: tray disposing portion E
- G: opening portion for manual insertion
- M: module of blood collection tube storage box

### Description of Embodiment

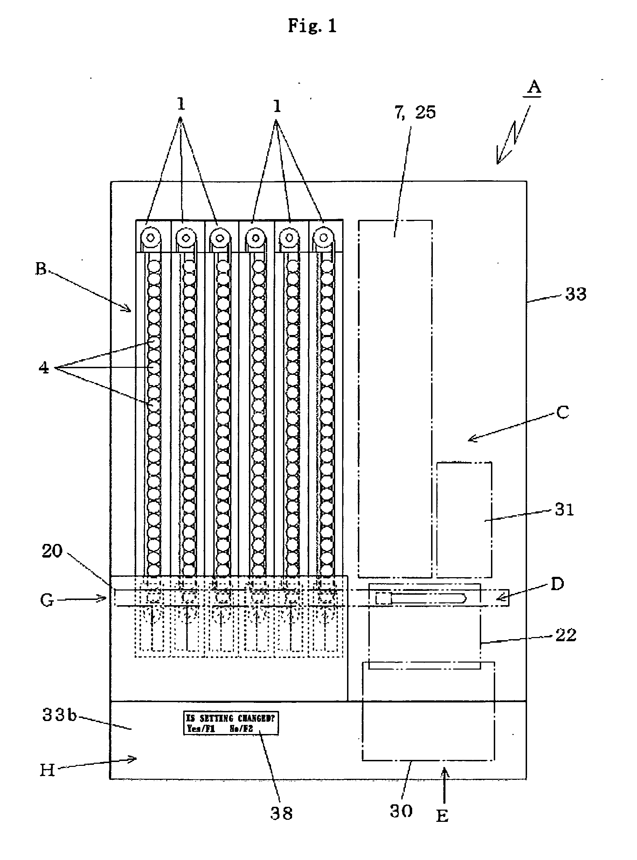

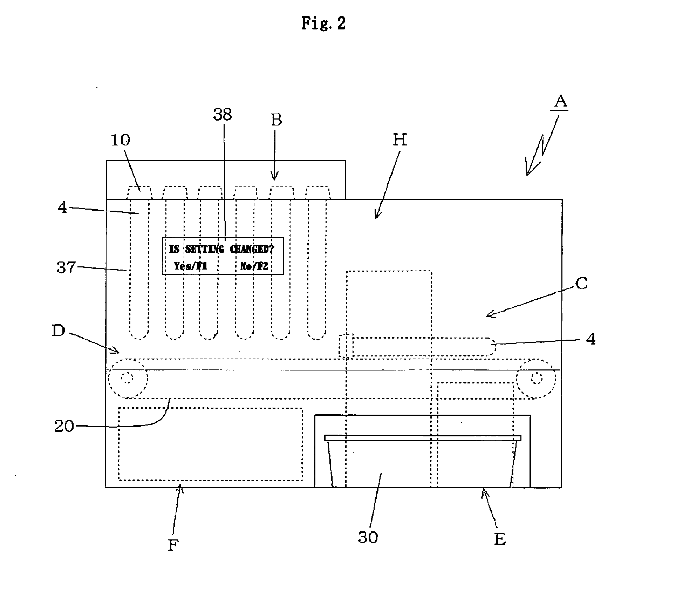

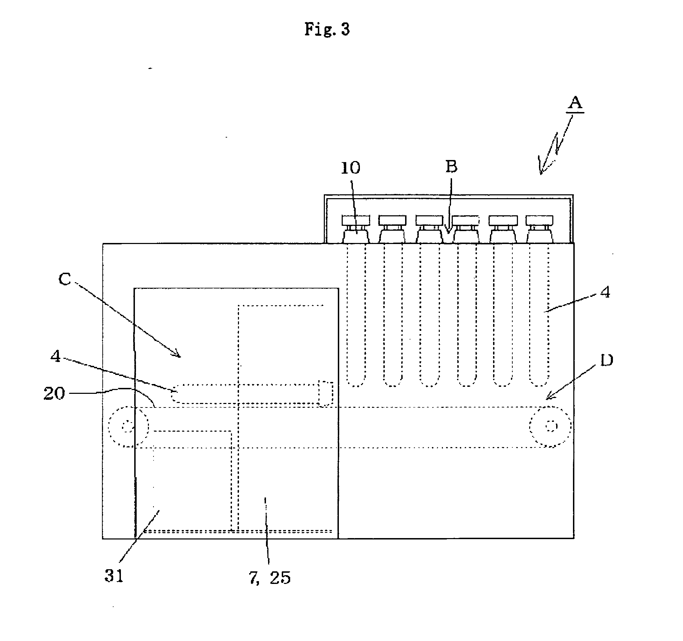

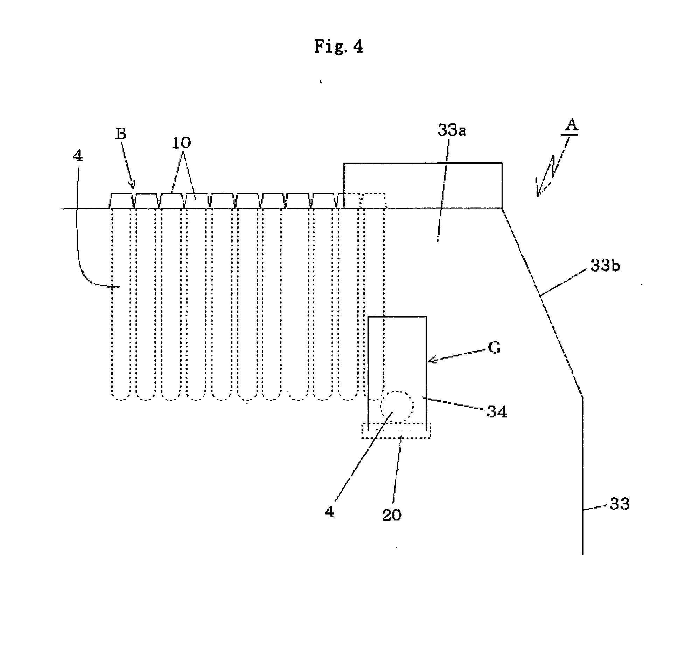

Hereinafter, a structure of the present invention is described as follows based on an embodiment illustrated in FIGS. 1 to 12. First, a layout of equipment and mechanisms constituting a blood collection tube preparing apparatus A is described with reference to FIGS. 1 to 4. FIGS. 1 to 4 illustrate an entire structure of the blood collection tube preparing apparatus A. FIG. 1 is a plan view, FIG. 2 is a front view, FIG. 3 is a back view, and FIG. 4 is a left-hand side view. As illustrated in the plan view of FIG. 1, in the blood collection tube preparing apparatus A, a mounting portion B for a plurality of blood collection tube storage boxes 1 is provided to occupy almost a left half region. In this embodiment, six storage boxes 1 are placed in parallel with each other in the form of modules, and the modules of the blood collection tube storage boxes 1 are detachably mounted to the mounting portion on an apparatus main body 33 side. On a right side of the storage box mounting portion B and inside the apparatus main body, a placing portion C for label printing/sticking means is provided. Further, below a blood collection tube discharging portion of each of the storage boxes 1 on the storage box mounting portion B, there is provided a placing portion D for a conveying belt 20 which crosses a front side of the placing portion C for the printing/sticking means from a left end side of the apparatus main body 33 in a direction orthogonal to each of the blood collection tube storage boxes 1 and reaches a right end side of the apparatus main body 33.

Further, a display placing portion H is provided to a front plate 33b on a front side of the apparatus main body 33. The display placing portion H is formed into an inclined surface so that information displayed on a display is easy to see for an operator standing in front of the apparatus. On the display placing portion H, a setting state display portion (display) 38 is provided.
The setting state display portion 38 displays a setting state of each component of the blood collection tube preparing apparatus, or displays guidance for changing the settings. For changing the settings, for example, the setting state display portion 38 displays the setting state in association with operation buttons of the blood collection tube preparing apparatus itself. Examples of the operation buttons include a restart button, a feed button, and function key buttons such as F1 to F3 keys. The setting state to be displayed includes states of during idling, waiting for initial communication command, already having received printing command, during maintenance mode, cover open, label shortage detection, head open detection, mount-sheet pressing roller open detection, sheet feeding motor error, pushdown motor error, positioning motor error, side positioning motor error, tube presence check error, empty storage box, stopper error, conveyor conveying time-out, conveyor jam detection, blood collection tube drop error, etc.

Further, as illustrated in the front view of FIG. 2, a tray disposing portion E is provided on a front side of the placing portion C for the printing/sticking means. A blood collection tube, to which the label having the printed patient information, blood collection test information, and the like has been stuck, is received into a tray 30 prepared in the tray disposing portion E for each patient. In the tray disposing portion E, optical sensors 36a and 36b (see FIG. 1) for detecting presence/absence of the tray 30 are arranged. As the sensors, there is illustrated a type in which the photodetector 36b detects light radiated from the projector 36a. However, the sensors may be a reflection type.

In addition, as illustrated in FIG. 4, an opening portion G which faces the placing portion D for the conveying belt 20 is provided on a left side plate 33a of the apparatus main body 33. The opening portion G is normally closed by an opening/closing door 34 pivotable about a lower end side thereof. Through the opening portion G, the blood collection tube is manually put onto the conveying belt 20. The opening/closing door 34 in an open state functions as a path for putting and guiding a blood collection tube 4. In this way, through the opening portion G, a rarely-used blood collection tube or a special-size blood collection tube can be individually supplied to the printing/sticking means.

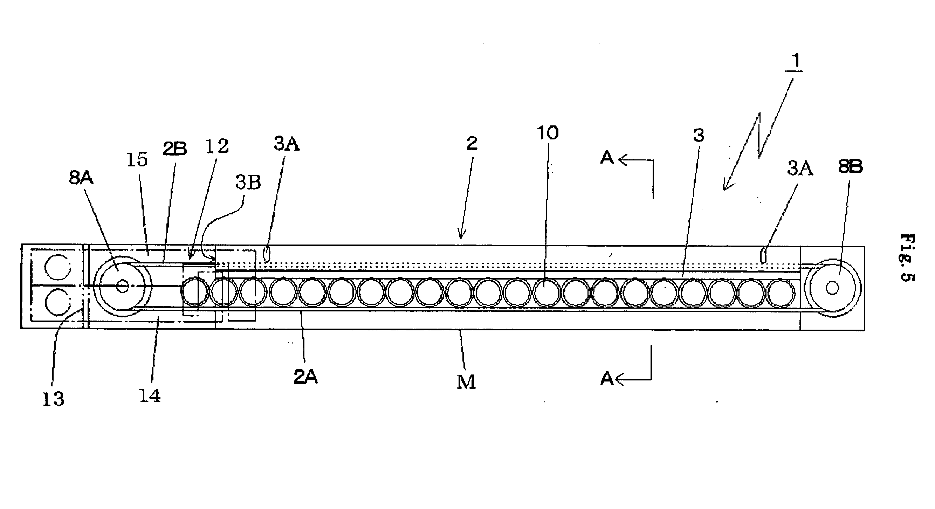

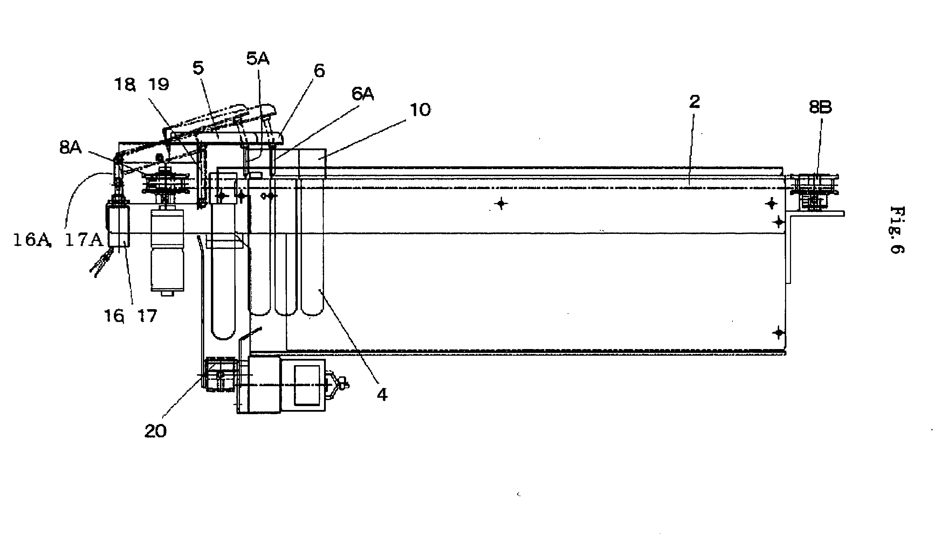

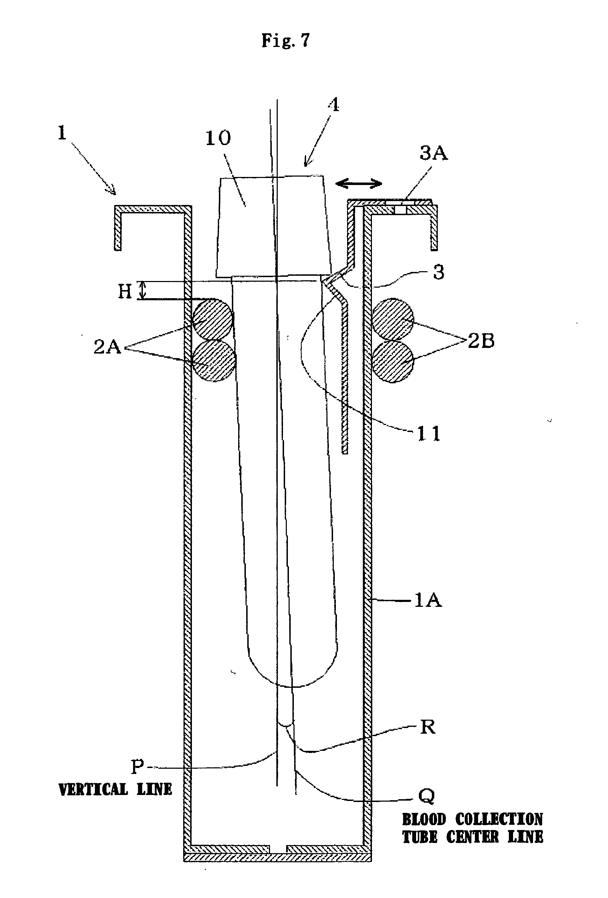

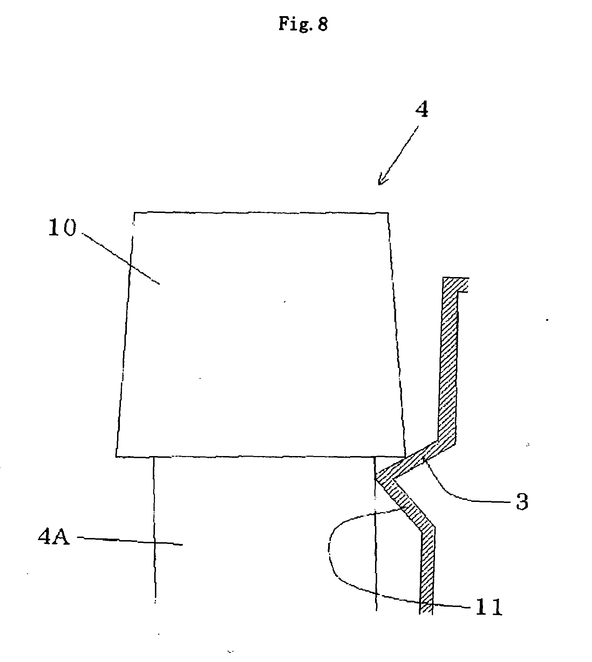

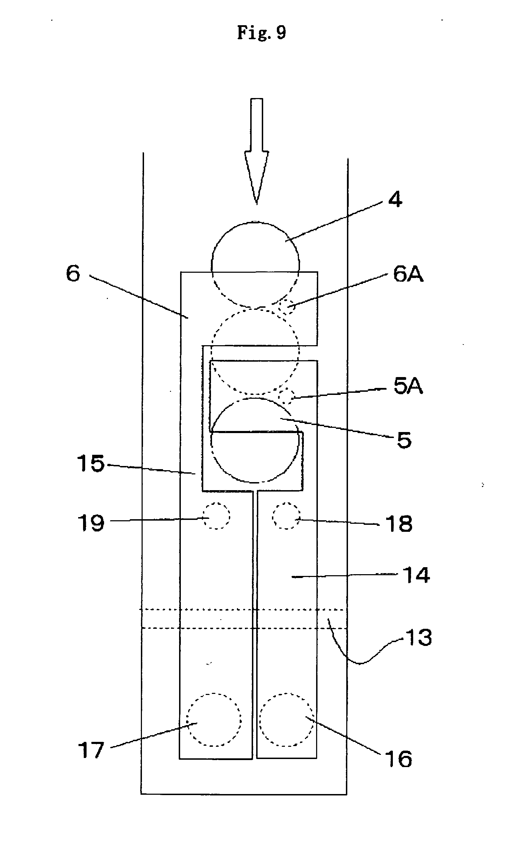

Next, each equipment and mechanism constituting the blood collection tube preparing apparatus A is individually described. First, each of the blood collection tube storage boxes 1 is described with reference to FIGS. 5 to 9. FIG. 5 is a plan view of the storage box 1, FIG. 6 is a side view thereof, FIG. 7 is a sectional view taken along the line A-A of FIG. 5, FIG. 8 is a partially enlarged view of FIG. 7, and FIG. 9 is an enlarged plan view of stoppers. As illustrated in the vertical sectional view of FIG. 7, the blood collection tube storage box 1 in this embodiment includes a casing 1A in which a blood collection tube conveying path (groove) having a U shape in cross-section is formed by bending a thin metal plate made of stainless steel, aluminum alloy, steel, or the like. Each component is coupled to the casing, to thereby structure the entire blood collection tube storage box 1 as one module M. Therefore, the blood collection tube storage box 1 can replaced and repaired in the module M unit through one-touch operation, and maintenance, replacement, and repair operations can be performed at remote locations through sending back only the module M for the operations. In addition, when substitutes for the module M are prepared in advance, it is unnecessary to stop a blood collection operation.

As illustrated in the plan view of FIG. 5, in the blood collection tube storage box 1, an endless transferring belt 2 is looped around a drive pulley 8A and a driven pulley 8B which are arranged in a laid posture at both ends of the blood collection tube storage box 1. In this embodiment, the transferring belt 2 is configured in such a manner that two round belts are arranged in the vertical direction. The number of round belts to be arranged in the vertical direction may be three. Reference numeral 2A denotes a forward-side transferring belt, and 2B denotes a backward-side transferring belt. The transferring belt 2 may be a flat belt.
As illustrated in the vertical sectional view of FIG. 7, in the transferring belt 2, the forward-side transferring belt 2A is arranged in the casing 1A of the blood collection tube storage box 1, and the backward-side transferring belt 2B is arranged outside the casing 1A. On the other hand, at a position opposed to the forward-side transferring belt 2A in the casing 1A, an inclined support plate 3 and a side surface support plate 11 are provided. In this embodiment, the inclined support plate 3 and the side surface support plate 11 are formed by bending one steel plate with sheet metal.

The inclined support plate 3 has a flat portion to be mounted so as to be joined to the upper end surface of the casing 1A, and is mounted so as to advance and retreat in a horizontal direction of FIG. 7 through a long hole 3A of the mounted flat portion. This is performed so as to handle the size of the tube diameter of the blood collection tube. Further, the inclined support plate 3 is in point contact with an edge portion of a lower end surface of a blood collection tube cap 10 so as to support the blood collection tube cap 10 in a suspended manner. Thus, the blood collection tube 4 is cantilever-supported in a suspended manner in the vertical direction by the point contact of the lower end surface edge portion of the cap 10 with the inclined support plate 3.

On the other hand, the side surface support plate 11 has a portion bent toward the inside of the casing 1A, and a tip end portion thereof abuts against a blood collection tube main body 4A while coming into point contact with the outer peripheral side surface of the blood collection tube main body 4A at a position opposed to the forward-side transferring belt 2A. Then, a difference in height H is provided between the upper end surface of the forward-side transferring belt 2A and a point where the side surface support plate 11 is in contact with the outer peripheral surface of the blood collection tube main body. Further, the distance in the horizontal direction between the forward-side transferring belt 2A and the point where the side surface support plate 11 is in contact with the outer peripheral surface of the blood collection tube main body is set to be smaller than the outer diameter dimension of the blood collection tube main body 4A. This interval is adjusted by allowing the inclined support plate 3 to reciprocate through the long hole 3A of the inclined support plate 3.
This enables a center line Q of the blood collection tube to be inclined by an angle R with respect to a vertical line P. Such setting makes it unnecessary to adjust the contact position between the blood collection tube 4 and the forward-side transferring belt 2A. More specifically, merely by inserting the blood collection tube 4 between the forward-side transferring belt 2A and the side surface support plate 11 so that the blood collection tube 4 is slightly inclined, the forward-side transferring belt 2A can be brought into abutment with the outer peripheral side surface of the blood collection tube 4 with some pressure force, which makes it unnecessary to adjust the interval between the forward-side transferring belt 2A and the blood collection tube 4.

Regarding the size of the blood collection tube 4, generally, there are blood collection tubes with a diameter of 13 mm and 16 mm for a biochemical test, an immunological test, a blood sugar test, a hematological test, a coagulation test, etc. There is a blood collection tube with a diameter of 8 mm for a sedimentation test. The blood collection tube 4 includes the tube-like blood collection tube main body 4A and the cap 10, and the lower end surface of the cap 10 has an outer diameter dimension larger than that of the blood collection tube main body 4A. As illustrated in FIGS. 7 and 8, the blood collection tube 4 is supported in the horizontal direction by the forward-side transferring belt 2A and the side surface support plate 11, and is supported in the vertical direction by the inclined support plate 3. Further, the inclined support plate 3 and the side surface support plate 11 both support the blood collection tube 4 by a point contact method, and the friction resistance between the respective members in these contact portions is very small.
Therefore, when a drive force is transmitted from the forward-side transferring belt 2A to the blood collection tube main body 4A, the blood collection tube 4 moves sliding over the inclined support plate 3 and the side surface support plate 11 in a point contact state while rotating to be conveyed. Thus, by setting the portion supporting the blood collection tube 4 to be a point contact, excluding the drive transmission portion with the conveying belt 2, the friction resistance can be reduced remarkably, and the blood collection tube 4 can be conveyed with a slight conveyance force. Thus, even a rubber cap tube, which cannot be conveyed conventionally, can be conveyed.

On the back surface side of the forward-side transferring belt 2A, as illustrated in FIG. 7, a member constituting the casing 1A of the module M also functions as a backup member so that the belt is not bent. By backing up the back surface side of the forward-side transferring belt 2A, the belt 2A can be joined and brought into abutment with the outer peripheral side surface of the blood collection tube 4 reliably.

On a terminal end 3B side of the inclined support plate 3 and the side surface support plate 11, a discharging portion 12 for the blood collection tube 4 is provided. The discharging portion 12 is provided in a space formed between the terminal end side of the inclined support plate 3 and the side surface support plate 11 and the pulley 8A. When one of the blood collection tubes 4 is transferred to the terminal end side of the inclined support plate 3 and the side surface support plate 11, the edge portion of the lower end surface of the cap 10 is disengaged from the inclined support plate 3 so that the blood collection tube 4 naturally falls down by its own weight.

Incidentally, at a position facing the discharging portion 12, a first stopper 5 and a second stopper 6 are provided. As illustrated best in the plan view of FIG. 9, the first stopper 5 and the second stopper 6 respectively have an arm 14 and an arm 15 which are pivotally and swingably supported by the same shaft 13 at middle portions of the arms, and on the lower surfaces on one end side of the arms 14 and 15, bar-like protrusions 5A and 6A are provided upright. Of portions of the first stopper 5 and the second stopper 6 provided with the bar-like protrusions 5A and 6A, portions bent into an L shape in plan view are arranged in a staggered manner so that one bend portion is fitted inside the other bend portion so as not to interfere with each other. The first stopper 5 is capable of moving close to and away from the front surface side of the cap of the leading blood collection tube 4 so as to abut against the same, and the second stopper 6 is capable of moving close to and away from a gap formed between the caps of the leading and second blood collection tubes 4.

Further, a rod of a solenoid 16 and a rod of a solenoid 17 are coupled onto lower surfaces on the other end side of the arms 14 and 15, respectively. As the solenoids 16 and 17 in this case, there are adopted ones having substantially the same outer diameter dimension as that of a ball-point pen for the purpose of downsizing. When the power source is turned ON, the rods 16A and 17A are operated to retract, to thereby swing the arms 14 and 15 to upward positions as illustrated by chain lines of FIG. 6. Note that, the solenoids 16 and 17 are normally set to perform the operation alternately. Return springs (tension springs) 18 and 19 are fixed between the pivot shaft 13 of the respective arms 14 and 15, and the bar-like protrusions 5A and 6A. This is adopted to return the arms 14 and 15 to horizontal postures due to tensile forces of the return springs 18 and 19 when the power source for the solenoids 16 and 17 is turned OFF. Further, the return springs 18 and 19 also function to prevent the falling blood collection tubes 4 from coming out to the front side.

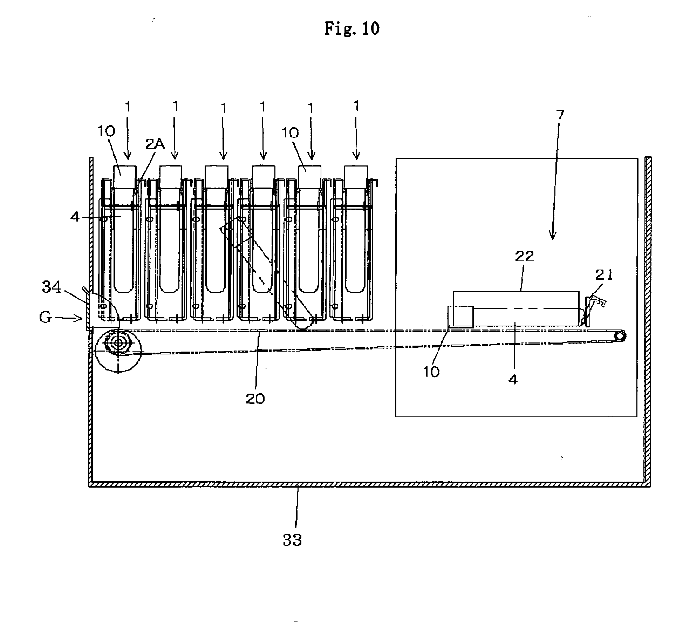

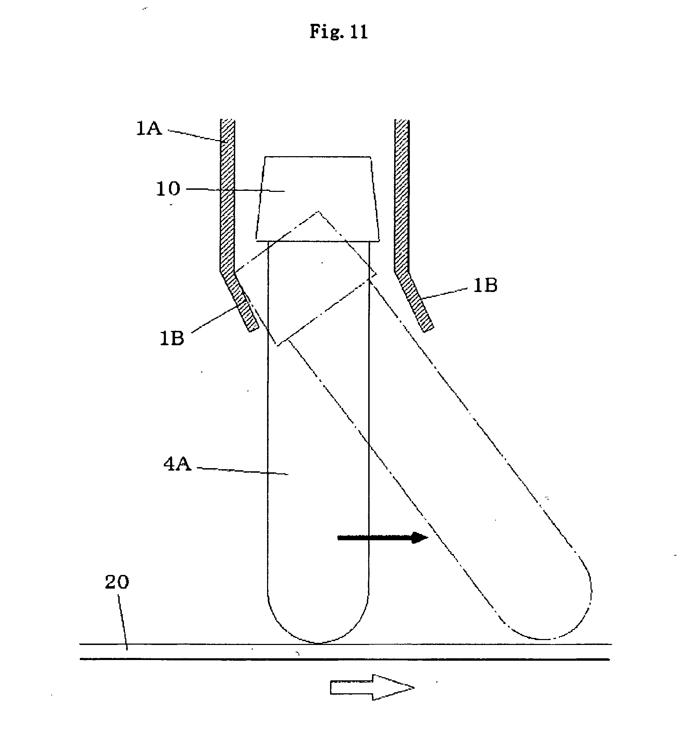

In this embodiment, as illustrated in FIGS. 1 and 10, the blood collection tube storage boxes 1 of a suspended type structured as described above are aligned in six rows. The one conveying belt 20 is provided below the discharging portion 12 in a direction orthogonal to a conveying direction of the transferring belt 2, and each of the blood collection tubes 4 supplied, while falling down, from each of the storage boxes 1 aligned in the six rows is conveyed by the conveying belt 20 from a left side of FIGS. 1 and 10 to a right side thereof. At this time, as illustrated in FIG. 11, only the bottom portion of the blood collection tube 4 advances while losing the balance by the conveying belt 20, and the cap 10 side of the blood collection tube 4 still remains in the casing 1A. Further, the exit side of the casing 1A has flaps 1B and 1B bent in the advancing direction, which enables the cap 10 side to be discharged last and the blood collection tube 4 to be directed always in a predetermined direction.

Note that, the conveying belt 20 is constituted by one continuous belt in the embodiment of FIG. 10. However, it is possible that two conveying belts are separately provided in a region for the storage boxes 1 and a region for label printing/sticking means 7 to allow each of the blood collection tubes 4 to be automatically transferred from one conveying belt to the other conveying belt. In this case, the blood collection tube preparing apparatus has a structure in which a storage box device and a label printing/sticking device are separately provided, and thus the storage box device can be used independently. Further, maintenance can be performed under a state in which the devices are separated from each other, which is convenient.

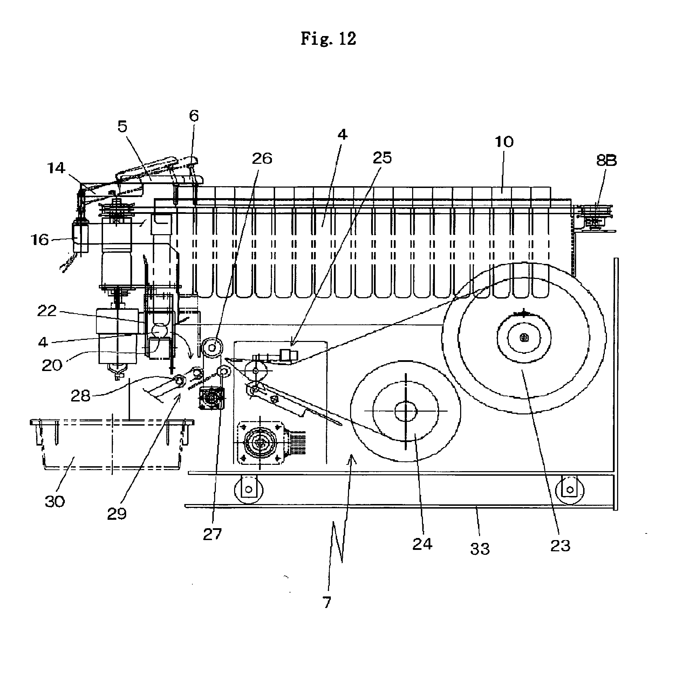

An arrival detecting plate 21 for the blood collection tubes 4 is provided on the terminal end side of the conveying belt 20. The arrival detecting plate 21, in a normal state, is on standby in an obliquely inclined posture as illustrated by chain lines of FIG. 10. When the blood collection tube 4 is conveyed, the arrival detecting plate 21 is pressed by the blood collection tube 4 to assume an upright posture as illustrated by solid lines, and simultaneously determines a stop position of the blood collection tube 4. The arrival detecting plate 21 has a portion bent into a C shape. When the arrival detecting plate 21 assumes the inclined standby state, light radiated from a detection sensor (not shown) including a projector and a photodetector, such as an infrared ray sensor or a photosensor, is blocked, and when the blood collection tube 4 has arrived so that the arrival detecting plate 21 assumes the upright state, the light radiated from the projector is received by the photodetector. In addition, as illustrated in FIGS. 10 and 12, at a side surface portion of the blood collection tube 4 at the stop position, there is provided a slider 22 for isolating the blood collection tube 4 from the other blood collection tubes, which reciprocates in a direction orthogonal to the conveying direction of the conveying belt 20 and the slider 22 pushes out the blood collection tube 4 from the conveying belt 20, to thereby supply the blood collection tube 4 to a sticking position for the label sticking device 7 while the blood collection tube 4 falls down.

Note that, orientation detecting means (not shown) for detecting an orientation of the blood collection tube 4 is provided just before the arrival detecting plate 21. This is structured to be able to detect the orientation of the blood collection tube 4 in the case where the cap 10 blocks the light radiated from the projector when the cap 10 improperly abuts against the arrival detecting plate 21 first so that the blood collection tube 4 is stopped.

As illustrated in FIG. 12, the label sticking device 7 includes a feeding roll 23 for feeding a label adhering onto a continuous sheet of release paper and a winding roll 24 for winding the sheet of release paper. The label and the sheet of release paper fed from the feeding roll 23 are moved while being folded back at an acute angle so that only the stiff label (which is more elastic and less foldable than the sheet of release paper) is peeled off from the sheet of release paper. The label is moved straight ahead as it is to be fed to a side of sticking means 29, and only the remaining sheet of release paper is wound by the winding roll 24. A printing device 25 for printing patient information and the like onto the label is provided on an upstream side of the releasing portion. The information to be printed is obtained from a database or a hard disk of a computer such as an upper medical system or a doctor's computer terminal by connecting to the computer or the computer terminal through an interface of a computer (CPU) having a built-in or externally-connected control portion of the blood collection tube preparing apparatus. In addition, in an advancing direction of the label peeled off from the sheet of release paper, the sticking means 29 including a drive roller 26, a support roller 27, and a pressure roller 28 capable of advancing/retreating is provided. Further, a disposing portion for the tray 30 is provided obliquely below the sticking means 29.

Next, operations of the blood collection tube preparing apparatus A structured as described above are described. First, the modules M of the blood collection tube storage boxes 1 for storing different kinds of blood collection tubes are set on six-row mounting portions in the storage box placing portion B provided on the upper surface of the apparatus main body 33. The modules M may be fixed with a screw fixing method or another fixing method such as one-touch clip method. Then, the blood collection tubes 4 are set manually in the groove (portion for storing the blood collection tubes 4) formed between the inclined support plate 3 and the side surface support plate 11 and the forward-side transferring belt 2A of each of the blood collection tube storage boxes 1, and another kind of blood collection tubes 4 are also set manually in another groove. When setting each of the blood collection tubes 4, the blood collection tube 4 only needs to be inserted into the above-mentioned groove from the bottom side thereof. In this way, each blood collection tube 4 is supported in a suspended manner in the vertical direction by the point contact of the lower end surface edge portion of the cap 10 with the inclined support plate 3, and the conveying belt 20 and the side surface support plate 11 sandwich the outer peripheral side surface of the blood collection tube main body 4A to support the blood collection tube main body 4A in the horizontal direction. Further, the conveying belt 20 comes into abutment with the outer peripheral side surface of the blood collection tube 4 with some pressure force.
In a state before starting the operation, the bar-like protrusion 5A of the first stopper 5 of the blood collection tube preparing apparatus abuts against the front surface side of the cap 10 of the leading blood collection tube 4, to thereby restrict the forward movement of the leading blood collection tube. Further, the bar-like protrusion 6A of the second stopper 6 enters a gap 31 formed between the cap 10 of the leading blood collection tube 4 and the cap 10 of the second blood collection tube 4, to thereby restrict the forward movement of the second blood collection tube 4.

The power source switch of the apparatus main body 33 is turned ON under this state, and the built-in or externally-connected computer (CPU) is operated to cause the control software to function. As a result, blood collection information for the patient to be subjected to blood collection is obtained from the upper computer or the doctor's computer terminal. Note that, the control portion (firmware) of the blood collection tube preparing apparatus A does not allow each equipment to operate under a state in which another tray 30 is not newly prepared for the patient after detecting that the tray 30 for the previous patient is removed from the tray disposing portion E. This is intended to prevent confusion which may occur when the blood collection tube for the next patient is put onto the tray 30 for the previous patient.

The control portion determines, based on the obtained information, a kind of the blood collection tube to be selected, and drives the drive pulley 8A of the blood collection tube storage box 1 storing the corresponding kind of the blood collection tubes 4 to rotate. Owing to drive of the drive pulley 8A, all of the blood collection tubes 4 of the storage box 1 receive a transferring force of the forward-side transferring belt 2A joined to the outer peripheral side surface thereof. Note that, under this state, the first stopper 5 and the second stopper 6 function to restrict the movement of the leading blood collection tube 4 and the second blood collection tube 4, and hence all of the blood collection tubes 4 remain stopped.

Next, the control portion of the blood collection tube preparing apparatus A turns ON the solenoid 16 of the first stopper 5 of the corresponding storage box 1. Thus, the rod of the solenoid 16 is operated to retract, to thereby cause the arm 14 to pivot on the pivot shaft 13 in a counterclockwise direction of FIG. 12. Accordingly, the bar-like protrusion 5A fixed on one end side of the arm 14 is moved away from the front surface side of the cap 10 of the leading blood collection tube 4, and cancels restriction of the leading blood collection tube 4. The leading blood collection tube 4 assumes a free state, and starts to advance from the stop position after being applied with the transferring force of the forward-side transferring belt 2A. After a while, the leading blood collection tube 4 reaches the discharging portion 12 on the terminal end side of the inclined support plate 3 to be disengaged from the inclined support plate 3, and hence, falls down onto the conveying belt 20 by its own weight. Then, the leading blood collection tube 4 is conveyed toward the printing/sticking portion with the bottom side of the blood collection tube 4 conveyed first.

Meanwhile, in the first stopper 5, when the solenoid 16 is turned OFF, the arm 14 is returned to an original horizontal state by the return spring 18. Then, the solenoid 17 of the second stopper 6 is turned ON, to thereby cause the arm 15 to pivot in a counterclockwise direction up to a position illustrated by chain lines of FIG. 12. Thus, the bar-like protrusion 6A cancels the joint with the cap 10 of the second blood collection tube 4. Accordingly, all of the remaining second and subsequent blood collection tubes 4 advance while receiving the drive force of the forward-side transferring belt 2A, and the front surface side of the cap 10 of the new leading blood collection tube 4 abuts against the bar-like protrusion 5A of the first stopper 5 so that the blood collection tube 4 is stopped. Finally, the solenoid 17 of the second stopper 6 is turned OFF and the arm 15 is returned to a horizontal state by the return spring 19, with the result that the bar-like protrusion 6A enters the gap formed between the cap 10 of the new leading blood collection tube 4 and the cap 10 of the second blood collection tube 4. The blood collection tube 4 is on standby in this state until the next supply instruction of the blood collection tube 4 is issued.

The conveying belt 20 starts to be driven at the same timing of turning ON the solenoid 16 of the first stopper 5. Thus, the blood collection tube 4 supplied while falling down by its own weight is conveyed by the conveying belt 20 from a leftward direction of FIG. 10 to a rightward direction thereof, to thereby press and urge the arrival detecting plate 21. Note that, even when the blood collection tube 4 is supplied onto the conveying belt 20 while falling down and assuming the upright state, the conveying belt 20 is driven from the leftward direction of FIG. 10 to the rightward direction thereof, and hence the blood collection tube 4 loses the balance. Further, the cap 10 is engaged with the flaps 1B and 1B, and is discharged last from the casing 1A. Thus, the bottom side of the blood collection tube is always conveyed first. The bottom of the blood collection tube 4 on the conveying belt 20 abuts against the arrival detecting plate 21 on the terminal end side of a conveying path so as to press the arrival detecting plate 21, and the blood collection tube 4 is stopped after swinging the arrival detecting plate 21 from the inclined posture to the upright posture.
Note that, at the stop position, an orientation detecting sensor (not shown) for the blood collection tube 4 detects the cap 10 of the blood collection tube 4. In the case of detecting the cap, it is detected that the cap is conveyed first so that the orientation of the blood collection tube is opposite, and hence an operator is informed of this circumstance through a warning issued by means of sound, a flashing signal of a light emitting device, or the like. In this case, the operator may continue the operation after taking out the blood collection tube 4 and changing the orientation.

The arrival detecting plate 21 is pressed and urged to the upright posture, and thus arrival detecting means such as a light emitting/receiving sensor functions. As a result, it is possible to detect that the blood collection tube 4 has been supplied. In response to the arrival detecting signal, the slider 22, which reciprocates in the direction orthogonal to the conveying direction of the conveying belt 20, starts to operate, and pushes out the blood collection tube 4, which have been arrived, in a lateral direction so that the blood collection tube 4 is supplied, while falling down, to the sticking means 29 side of the label sticking device 7. In the sticking means 29, the pressure roller 28 is at a retreat position, and, when receiving the blood collection tube 4, the pressure roller 28 advances to press and urge the blood collection tube 4 toward the drive roller 26 and the support roller 27. Then, the drive force of the drive roller 26 is transmitted to the blood collection tube 4 so that the blood collection tube 4 is rotated in a circumferential direction.

Meanwhile, in the label printing device 25, necessary items including blood collection information, such as a patient ID, a test item, a kind of blood collection tube, and a blood collection volume, obtained from the upper computer, a doctor's computer terminal, or the like are printed on the label fed from the feeding roller 23. The contents to be printed may be confirmed on a CPU monitor. As illustrated in FIG. 10, the sheet of release paper, to which the printed label adheres, is moved while being folded back at an acute angle, and thus only the stiff label is moved straight ahead at it is, to thereby be peeled off from the sheet of release paper naturally. Then, the peeled label enters between the drive roller 26 and an outer peripheral surface of the blood collection tube 4, and is wound around and stuck onto the outer peripheral surface of the blood collection tube 4. When the pressure roller 28 is returned so as to retreat obliquely downward (to a left lower side of FIG. 12), the blood collection tube 4 with the label stuck thereon is released from the sticking portion, and falls down by its own weight to be received in the tray 30.

With the above-mentioned procedure, preparation for one blood collection tube of one tube kind is completed. Thereafter, if another blood collection tube is required, the storage box 1 corresponding to the kind of the another blood collection tube is driven, and the above-mentioned operations are sequentially repeated. Thus, the blood collection tube 4 is received in the tray 30. Further, if there is a blood collection tube of a tube kind that is not stored in the six storage boxes 1 and is to be manually inserted, the blood collection tube may be individually supplied onto the conveying belt 20 through the opening portion G for manual insertion formed in a left side surface of the apparatus main body, and be received in the tray 30 after being subjected to label sticking. In this way, when preparation is completed for blood collection tubes of all tube kinds required based on the information obtained from the upper computer, the doctor's computer terminal, or the like, preparation is completed for blood collection tubes used to perform blood collection on one patient. Thereafter, blood collection tubes for another patient may be prepared in the similar way.

### Industrial Applicability

Incidentally, the present invention is not limited to the above-mentioned embodiments, and may be modified appropriately. For example, description is made on the case where the round belt is adopted as the transferring belt 2 used for the blood collection tube storage box 1. However, a normal flat belt may be adopted. In this case, as the belt to be used, a rubber belt is suitable because a friction resistance of the rubber belt with the outer peripheral side surface of the blood collection tube is large so that the drive force of the belt is transmitted. Further, description is made on the case where six blood collection tube storage boxes 1 are aligned. However, the number of the blood collection tube storage boxes may be below six, for example, one, two, or three, and may be above six. It is possible to arbitrarily set the number of the storage boxes depending on the purpose. The blood collection tube storage boxes 1 can be set onto the apparatus main body 33 in the form of modules, and hence it is possible to easily change the sizes of the blood collection tubes even after shipment . Further, as the modules of the blood collection tube storage boxes 1, for example, a plurality of kinds of modules, such as modules of boxes of ten, fifteen, and twenty tubes, can be prepared so as to store the various numbers of blood collection tubes, and the plurality of kinds of modules can be easily replaced.

Further, description is made on the case where the rods retract when the solenoids 16 and 17 are turned ON. However, the rods may reciprocate, i.e., protrude and retract. In this case, it is possible to omit the return springs 18 and 19.

Still further, it is possible that two conveying belts are provided continuously through partitioning the conveying belt 20 into the region for the storage boxes 1 and the region for the label sticking device 7, to thereby allow each of the blood collection tubes 4 to be automatically transferred from one conveying belt to the other conveying belt. In this case, it is possible to separate external covers constituting the casing of the blood collection tube preparing apparatus into the region for the storage boxes 1 and the region for the label sticking device 7, and hence an individual maintenance operation can be performed in each of the regions. Further, a blood collection tube storage box device and the label sticking device 7 may be structured separately from each other. In this case, it is possible to sell the blood collection tube storage box device alone, and to structure the blood collection tube preparing apparatus through coupling together the blood collection tube storage box device and the label sticking device as an optional product.

Further, the bar-like protrusions 5A and 6A of the first and second stoppers 5 and 6 may be strip-like plate members having substantially the same curved surface as that of the outer peripheral surface of the blood collection tube cap.

## Claims

1. A blood collection tube storage box, comprising:
an endless transferring belt that is looped around pulleys provided on both end sides of the endless transferring belt and is driven to rotate in a horizontal direction, the endless transferring belt being brought into abutment with an outer peripheral side surface of a blood correction tube to transfer the blood collection tube;
a side surface support plate having an end surface partially brought into point contact with an outer peripheral side surface of the blood collection tube on an opposite side of the endless transferring belt to support the blood collection tube;
an inclined support plate brought into point contact with a lower end surface edge of a cap of the blood collection tube on the opposite side of the endless transferring belt to support the blood correction tube in a suspended manner,
the side surface support plate and the inclined support plate being positioned between reciprocating belts of the endless transferring belt and above an upper end of the endless transferring belt;
a discharging portion through which the blood collection tube falls down by an own weight of the blood collection tube to be discharged, the discharging portion being provided at a position at which the blood collection tube passes a terminal end side of the inclined support plate that supports the blood collection tube in the suspended manner;
a first stopper capable of moving close to and away from a front surface of a cap of a leading blood collection tube;
a second stopper capable of moving close to and away from a gap from above, the gap being formed between the cap of the leading blood collection tube and a cap of a second blood collection tube,
the first stopper and the second stopper being provided on the terminal end side of the inclined support plate;
two arms which are pivotally supported by the same shaft, and to which the first stopper and the second stopper are fixed on distal end sides of the two arms, respectively; and
solenoids coupled on trailing end sides of the two arms, respectively.

2. A blood collection tube storage box, comprising:
an endless transferring belt that is looped around pulleys provided on both end sides of the endless transferring belt and is driven to rotate in a horizontal direction, the endless transferring belt being brought into abutment with an outer peripheral side surface of a blood correction tube to transfer the blood collection tube;
a side surface support plate having an end surface partially brought into point contact with an outer peripheral side surface of the blood collection tube on an opposite side of the endless transferring belt to support the blood collection tube;
an inclined support plate brought into point contact with a lower end surface edge of a cap of the blood collection tube on the opposite side of the endless transferring belt to support the blood correction tube in a suspended manner,
the side surface support plate and the inclined support plate being positioned between reciprocating belts of the endless transferring belt and above an upper end of the endless transferring belt;
a discharging portion through which the blood collection tube falls down by an own weight of the blood collection tube to be discharged, the discharging portion being provided at a position at which the blood collection tube passes a terminal end side of the inclined support plate that supports the blood collection tube in the suspended manner;
a first stopper capable of moving close to and away from a front surface of a cap of a leading blood collection tube;
a second stopper capable of moving close to and away from a gap from above, the gap being formed between the cap of the leading blood collection tube and a cap of a second blood collection tube,
the first stopper and the second stopper being provided on the terminal end side of the inclined support plate;
two arms which are pivotally supported by the same shaft, and to which the first stopper and the second stopper are fixed on distal end sides of the two arms, respectively; and
solenoids coupled on trailing end sides of the two arms, respectively,
the endless transferring belt, the inclined support plate, the discharging portion for the blood collection tube, the first stopper, the second stopper, the two arms, and the solenoids being accommodated in one casing, to thereby form a module.

3. A blood collection tube preparing apparatus, comprising:
an endless transferring belt that is looped around pulleys provided on both end sides of the endless transferring belt and is driven to rotate in a horizontal direction, the endless transferring belt being brought into abutment with an outer peripheral side surface of a blood correction tube to transfer the blood collection tube;
a side surface support plate having an end surface partially brought into point contact with an outer peripheral side surface of the blood collection tube on an opposite side of the endless transferring belt to support the blood collection tube;
an inclined support plate brought into point contact with a lower end surface edge of a cap of the blood collection tube on the opposite side of the endless transferring belt to support the blood correction tube in a suspended manner,
the side surface support plate and the inclined support plate being positioned between reciprocating belts of the endless transferring belt and above an upper end of the endless transferring belt;
a discharging portion through which the blood collection tube falls down by an own weight of the blood collection tube to be discharged, the discharging portion being provided at a position at which the blood collection tube passes a terminal end side of the inclined support plate that supports the blood collection tube in the suspended manner;
a first stopper capable of moving close to and away from a front surface of a cap of a leading blood collection tube;
a second stopper capable of moving close to and away from a gap from above, the gap being formed between the cap of the leading blood collection tube and a cap of a second blood collection tube,
the first stopper and the second stopper being provided on the terminal end side of the inclined support plate;
two arms which are pivotally supported by the same shaft, and to which the first stopper and the second stopper are fixed on distal end sides of the two arms, respectively;
solenoids coupled on trailing end sides of the two arms, respectively;
a conveying belt for the blood collection tube, the conveying belt being provided below the discharging portion;
label printing/sticking means provided on a terminal end side of the conveying belt; and
a tray disposing portion for receiving the blood collection tube, the tray disposing portion being provided in a vicinity of a delivery portion of the label printing/sticking means.

4. A blood collection tube preparing apparatus, comprising:
a plurality of blood collection tube storage boxes, each of the plurality of blood collection tube storage boxes comprising:
an endless transferring belt that is looped around pulleys provided on both end sides of the endless transferring belt and is driven to rotate in a horizontal direction, the endless transferring belt being brought into abutment with an outer peripheral side surface of a blood correction tube to transfer the blood collection tube;
a side surface support plate having an end surface partially brought into point contact with an outer peripheral side surface of the blood collection tube on an opposite side of the endless transferring belt to support the blood collection tube;
an inclined support plate brought into point contact with a lower end surface edge of a cap of the blood collection tube on the opposite side of the endless transferring belt to support the blood correction tube in a suspended manner,
the side surface support plate and the inclined support plate being positioned between reciprocating belts of the endless transferring belt and above an upper end of the endless transferring belt;
a discharging portion through which the blood collection tube falls down by an own weight of the blood collection tube to be discharged, the discharging portion being provided at a position at which the blood collection tube passes a terminal end side of the inclined support plate that supports the blood collection tube in the suspended manner;
a first stopper capable of moving close to and away from a front surface of a cap of a leading blood collection tube;
a second stopper capable of moving close to and away from a gap from above, the gap being formed between the cap of the leading blood collection tube and a cap of a second blood collection tube,
the first stopper and the second stopper being provided on the terminal end side of the inclined support plate;
two arms which are pivotally supported by the same shaft, and to which the first stopper and the second stopper are fixed on distal end sides of the two arms, respectively; and
solenoids coupled on trailing end sides of the two arms, respectively,
the endless transferring belt, the blood collection tube supporting member, the discharging portion for the blood collection tube, the first stopper, the second stopper, the two arms, and the solenoids being accommodated in a module serving as one casing, the plurality of blood collection tube storage boxes being detachably and respectively received, in a form of the modules, in a plurality of lanes aligned in parallel to each other;
a conveying belt provided below a discharging section ranging over a region of the discharging portion of the each of the plurality of blood collection tube storage boxes;
label printing/sticking means provided on a terminal end side of the conveying belt; and
a tray disposing portion for receiving the blood collection tube, the tray disposing portion being provided in a vicinity of a delivery portion of the label printing/sticking means.

5. A blood collection tube preparing apparatus according to claim 3 or 4, further comprising an opening portion which is formed in a vicinity of the discharging portion of one of the plurality of blood collection tube storage boxes situated on a most upstream side of the conveying belt, and which allows the blood collection tube to be manually inserted onto the conveying belt through the opening portion.

6. A blood collection tube preparing apparatus according to any one of claims 3 to 5, further comprising a setting state display portion provided on an outer surface of a blood collection tube preparing apparatus main body, for displaying a setting state of the blood collection tube preparing apparatus and displaying guidance for changing settings in association with each operation switch.
